# EUROPÄISCHE PATENTSCHRIFT

(11) **EP 3 110 496 B1**
(45) Veröffentlichungstag und Bekanntmachung des Hinweises auf die Patenterteilung: **22.01.2020**
(21) Anmeldenummer: 15707081.4
(22) Anmeldetag: 24.02.2015
(51) Int. Cl.: A61M 1/36, A61M 39/16, F16B 39/30, A61M 39/10

(54) **SCHRAUBVERBINDER FÜR MEDIZINISCHE SCHLAUCHSYSTEME UND MEDIZINISCHE SCHLAUCHSYSTEME MIT SCHRAUBVERBINDER**
SCREW CONNECTOR FOR MEDICAL HOSE SYSTEMS AND MEDICAL HOSE SYSTEMS WITH SCREW CONNECTOR
RACCORD FILETÉ POUR SYSTÈMES DE TUYAUX MÉDICAUX ET SYSTÈMES DE TUYAUX MÉDICAUX ÉQUIPÉS D'UN RACCORD FILETÉ

(30) Priorität: 27.02.2014 DE 102014002649
(43) Veröffentlichungstag der Anmeldung: 04.01.2017
(73) Patentinhaber: Fresenius Medical Care Deutschland GmbH, 61352 Bad Homburg (DE)
(72) Erfinder: LAPP, Uwe, 35510 Butzbach (DE); LEICK, Lothar, 66663 Merzig (DE); MÜLLER, Ralf, 61350 Bad Homburg (DE); URBAN, Peter, 66649 Oberthal (DE)
(74) Vertreter: Rau, Jenspeter
(86) Internationale Anmeldenummer: PCT/EP2015/053789
(87) Internationale Veröffentlichungsnummer: WO 2015/128310

(56) Entgegenhaltungen:
- GB-A- 555 123
- JP-A- 2005 000 466
- US-A- 5 676 270
- US-A1- 2011 028 913
- US-B1- 6 381 928

## Beschreibung

### Technisches Gebiet

Die Erfindung betrifft einen Schraubverbinder für medizinische Schlauchsysteme und ein medizinisches Schlauchsystem mit Schraubverbinder sowie die Sterilisation solcher medizinischen Schlauchsysteme mittels Dampfsterilisierung und/oder Gassterilisierung. Es sind eine Vielzahl von Verbindern für medizinische Schlauchsysteme bekannt, insbesondere sogenannte Luer-Lock Konnektoren. Medizinische Schlauchsysteme kommen beispielsweise in der Infusionstechnik und in der extrakorporalen Blutbehandlung zum Einsatz und müssen für diese Anwendungen steril sein.

### Hintergrund

Zur Gewährleistung der Sterilität werden medizinische Schlauchsysteme für die Infusionstechnik und die extrakorporale Blutbehandlung in einer geeigneten geschlossenen Umhüllung verpackt und in dieser Verpackung sterilisiert. Die Sterilisierung kann beispielsweise als Dampfsterilisierung und/oder als Gassterilisierung erfolgen. Die vorgenannten Sterilisierverfahren erfordern, dass Gase und/oder Heißdampf zuverlässig an alle äußeren und inneren Oberflächen des medizinischen Schlauchsystems gelangen können.

Die Schrift DE 35 15 665 C1 offenbart einen Verschlussstopfen zum dichten Verschließen von Anschlüssen in der Medizintechnik, insbesondere von Luer-Lock-Anschlüssen. Seine beiden Verbindungsteile gestatten wahlweise ein loses Aufsetzen, bei dem ein Gasdurchtritt noch möglich ist, als auch das dichte Verschließen des Anschlusses. Die lose aufgesetzte Stellung und die Schließstellung sind durch einen Anschlag getrennt, der unter elastischer Verformung überwindbar ist. Dazu wird vorgeschlagen, den Anschlag mittels zwei innerer radialer Ringrippen vor dem Innengewinde des Verschlusstopfens und zwei äußeren Flügeln am komplementären Verbindungsteil auszuführen.

Ein Nachteil dieses bekannten Verschlussstopfens ist, dass die zwei inneren radialen Ringrippen nur durch eine zur Rotationsachse des Verschlussstopfens axial wirkende Druckkraft auf die Verbindungsteile einzeln und nacheinander überwunden werden können. Wenn die Druckkraft nicht exakt axial wirkt, kann es zum Verkanten kommen. Demzufolge muss der Anwender die Verbindungsteile sehr genau ausrichten, was dadurch erschwert wird, dass die Ringrippen unmittelbar vor dem Innengewinde des Verschlusstopfens angeordnet sind.

Beim Aufwenden einer nicht genau axial wirkenden Kraft oder einer zu kleinen axialen Kraft kann der Verschlusstopfen abfallen und im ungünstigsten Fall sogar auf den Boden fallen, so dass er nicht mehr steril ist. Daher ist der bekannte Verschlussstopfen unsicher und umständlich in der Handhabung.

Insgesamt führt das dazu, dass das Klinikpersonal mit fehleranfälligen und/oder umständlichen Prozeduren belastet wird.

Die Schrift WO 99/20376 A1 schlägt ein Verfahren zum Spülen eines Blutschlauchsatzes für die extrakorporale Blutbehandlung vor, wobei vorgeschlagen wird, den arteriellen Patientenkonnektor und den venösen Patientenkonnektor bereits während der Produktion des Blutschlauchsatzes mit einem Verbinder zu verbinden und in dieser verbundenen Form zu sterilisieren.

Die Schrift WO 96/40320 A1 schlägt ein weiteres Verfahren zum Spülen eines Blutschlauchsatzes für die extrakorporale Blutbehandlung vor, wobei vorgeschlagen wird, den arteriellen Patientenkonnektor und den venösen Patientenkonnektor beim Spülen des Blutschlauchsatzes über einen Y-Verbinder mit einem Ablauf zu verbinden. Dazu wird vorgeschlagen, dass der verpackte extrakorporale Blutschlauchsatz bereits den mit dem arteriellen Patientenkonnektor und dem venösen Patientenkonnektor verbundenen Y-Verbinder enthält.

Die Schrift WO 2010/121819 A1 der Anmelderin der vorliegenden Patentanmeldung offenbart eine Blutkassette mit einem Blutschlauchsatz für die extrakorporale Blutbehandlung mit einer arteriellen Patientenleitung und einer venösen Patientenleitung. Des Weiteren offenbart die Schrift WO 2010/121819 A1 auch ein Verfahren zum Spülen eines extrakorporalen Blutkreislaufs, wobei die Patientenkonnektoren der arteriellen Patientenleitung und der venösen Patientenleitung mittels eines T-Verbinders an dem Rinse-Port der Dialysemaschine angeschlossen wird. Auf den Inhalt der Schrift WO 2010/121819 A1 wird in der vorliegenden Patentanmeldung vollumfänglich Bezug genommen.

Es sind Kegelverbindungen mit einem 6 % (Luer) Kegel für Spritzen, Kanülen und bestimmte andere medizinische Geräte, insbesondere verriegelbare Kegelverbindungen bekannt, die auch als Luer-Lock-Verbindungen bezeichnet werden.
Die Schrift JP 2005 000466 A beschreibt einen Spiel-Verhinderungsmechanismus für einen Verschlussring eines medizinischen Luer-Konnektors, mit dem die Sicherheit des Verschlussrings bei der Anwendung des medizinischen Luer-Konnektors aufrechterhalten werden kann.
Die Schrift US 2011/028913 A1 beschreibt ein selbstsicherndes Gewinde für die Montage einer Spritzendichtung am Plunger einer medizinischen Spritze, wobei die Gewindeverbindung zwischen Plunger und Dichtung gegen unbeabsichtigtes Lösen gesichert werden soll.
Die Schrift US 6 381 928 B1 beschreibt einen Originalitätsverschluss mit selbstsicherndem Gewinde für einen Behälter.
Die Schrift GB 555 123 A beschreibt selbstsichernde Schraubelemente.
Die Schrift US 5 676 270 A beschreibt einen selbstsichernden Behälterverschluss mit Schraubgewinde.

### Zusammenfassung der Erfindung

Es ist daher eine Aufgabe der vorliegenden Erfindung mindestens einen der genannten Nachteile zu überwinden und einen verbesserten Schraubverbinder für medizinische Schlauchsysteme bereitzustellen.

Diese Aufgabe wird gelöst mit den Merkmalen der unabhängigen Patentansprüche 1, 7 und 12. Vorteilhafte Ausführungsformen sind in den Unteransprüchen angegeben.

Die vorliegende Erfindung stellt einen Schraubverbinder für ein medizinisches Schlauchsystem bereit mit mindestens einem ersten Verbindungselement mit einem ersten Lumen und einem Außengewinde
und mindestens einem zweiten Verbindungselement mit einem zweiten Lumen und einer elastisch verformbaren Überwurfmutter mit einem Innengewinde. Das Innengewinde und das Außengewinde sind
konfiguriert um miteinander verschraubt zu werden, wobei das Außengewinde mindestens eine erste Ausformung aufweist und das Innengewinde mindestens eine zweite Ausformung aufweist. Das Innengewinde und das Außengewinde sind konfiguriert zum gegenseitigen Verschrauben unter elastischer Verformung der Überwurfmutter mittels
der ersten Ausformung in mindestens einem vorgegebenen ersten Drehwinkelbereich, ohne dass die erste Ausformung in die zweite Ausformung einrastet. Das Innengewinde und das Außengewinde sowie
die erste Ausformung und die zweite Ausformung sind konfiguriert zum Einrasten der ersten Ausformung in die zweite Ausformung wenn das Innengewinde und das Außengewinde in einem vorgegebenen zweiten Drehwinkelbereich verschraubt sind.

Dabei sind das erste Verbindungselement und das zweite Verbindungselement jeweils vorteilhaft konfiguriert um beim Verschrauben des Innengewindes mit dem Außengewinde in dem zweiten Drehwinkelbereich zwischen einer äußeren Umgebung des Schraubverbinders und einem zumindest durch das erste Lumen gebildeten Inneren des Schraubverbinders gasdurchlässig zu sein, was nachfolgend als halboffene Sterilisierstellung bezeichnet wird.

Der Begriff des Schraubverbinders ist im Zusammenhang mit der vorliegenden Erfindung nicht eingeschränkt auf eine bestimmte Ausführungsform der flüssigkeitsdichten Verbindung eines ersten Lumens mit einem zweiten Lumen. Die flüssigkeitsdichte Verbindung, insbesondere durch Dichtungsflächen, kann unabhängig von der Verschraubung sein und/oder zusätzlich zu der Verschraubung vorliegen. Die Verschraubung kann insbesondere eine zusätzliche Sicherung und/oder Verriegelung einer flüssigkeitsdichten Verbindung eines ersten Lumens mit einem zweiten Lumen gegen unbeabsichtigtes Lösen umfassen.

Unter einer halboffenen Sterilisierstellung kann in manchen Ausführungsformen verstanden werden, dass die beiden komplementären Hälften eines Luer-Lock-Verbinders nicht dichtend verbunden sind sondern dass zwischen den komplementären Konusflächen des Luer-Lock-Verbinders ein definierter Spalt eingestellt ist. Dadurch kann beim Dampf- und/oder Gassterilisieren der Dampf- und/oder das Gas durch den Spalt auch zwischen die komplementären Hälften und in das jeweilige Innere der komplementären Hälften des Luer-Lock-Verbinders gelangen. Der Begriff "halboffen" wird verwendet um deutlich zu machen, dass die beiden komplementären Hälften des Luer-Lock-Verbinders jeweils zumindest teilweise durch die jeweils komplementäre Hälfte des Luer-Lock-Verbinders abgedeckt sind. Das hat den Vorteil, dass für beide komplementären Hälften des Luer-Verbinders trotz des definierten Spalts ein Berührschutz gewährleistet ist, ohne dass dafür zusätzliche Berührschutzkappen erforderlich sind.

In manchen Ausführungsformen kann die halboffene Sterilisierstellung den Vorteil aufweisen, dass die beide komplementären Hälften des Luer-Lock-Verbinders bereits vormontiert sind und für die Anwendung nur noch dichtend verbunden und durch Zuschrauben gesichert werden müssen. Solche Ausführungsformen sind besonders anwenderfreundlich, weil dem Anwender der Arbeitsgang des Konnektierens des Luer-Verbinders erleichtert und/oder zumindest teilweise abgenommen wird.

In einer Ausführungsform sind das erste Verbindungselement und das zweite Verbindungselement jeweils als komplementäre Hälfte eines gemeinsamen Luer-Lock Verbinders ausgebildet. Das Innengewinde und das Außengewinde sind jeweils als komplementäre Gewinde des Luer-Lock Verbinders ausgebildet und dienen als zusätzliche Sicherung des Luer-Konus gegen unbeabsichtigtes Lösen. Die komplementären Gewinde des Luer-Lock Verbinders sind zumindest hinsichtlich Gewindeform, Gewindedurchmesser, Gewindesteigung und Gewindelänge kompatibel ausgebildet und so zum gegenseitigen Verschrauben konfiguriert und vorgesehen.

In einer Ausführungsform des Schraubverbinders ist das erste Lumen als weibliche Hälfte des Luer-Konnektors ausgebildet und das zweite Lumen ist als männliche Hälfte des Luer-Konnektors ausgebildet.

Die Gewindemutter des Schraubverbinders ist vorzugsweise kompatibel mit den komplementären Hälften standardisierter Verbinder, weil die zweite Ausformung auf den Gewindeflanken des Innengewindes der Gewindemutter gegenüber den komplementären Hälften standardisierter Verbinder nicht störend wirkt, so dass beispielsweise das Verschrauben von standardisierten Luer-Lock Konnektoren an den Schlauchleitungsschnitten von Dialysenadeln, Kanülen, Kathetern oder an Ports mit der erfindungsgemäßen Gewindemutter nicht beeinträchtigt wird.

Das Außengewinde weist bevorzugt eine erste Ausformung auf, die eine lokale Erhebung auf dem Kerndurchmesser des Außengewindes zwischen zwei benachbarten Gewindeflanken ist. Dadurch ergibt sich im Bereich der Erhebung lokal ein vergrößerter Kerndurchmesser des Außengewindes.

Das Innengewinde weist bevorzugt eine zweite Ausformung auf, die eine lokale Freistellung an der Gewindeflanke des Innengewindes ist.

In einer Ausführungsform ist die lokale Freistellung als eine im Wesentlichen in axialer Richtung des Innengewindes verlaufende Nut in einer Gewindeflanke des Innengewindes ausgestaltet und die lokale Erhebung ist als eine im Wesentlichen in axialer Richtung des Außengewindes verlaufende Rippe zwischen zwei benachbarten Gewindeflanken des Außengewindes ausgestaltet.

In einer anderen Ausführungsform umfasst die erste Ausformung mehrere voneinander beabstandete lokale Erhebungen auf dem Kerndurchmesser des Außengewindes zwischen zwei benachbarten Gewindeflanken. Der Kerndurchmesser des Außengewindes ist dann jeweils im Bereich jeder der lokalen Erhebungen vergrößert. Das Innengewinde weist bevorzugt mehrere zweite Ausformungen auf, die lokale Freistellungen an der Gewindeflanke des Innengewindes sind. Die Anzahl der lokalen Erhebungen kann der Anzahl der lokalen Freistellungen entsprechen.

Im Einklang mit der Lehre der vorliegenden Erfindung ist die Mutter konfiguriert zur elastischen Verformung durch die erste Ausformung in einem vorgegebenen ersten Drehwinkelbereich, in dem die erste Ausformung nicht in die zweite Ausformung einrastet. Dazu kann der Durchmesser der Mutter beim Verdrehen in einem ersten Drehwinkelbereich elastisch aufgeweitet werden, wenn eine im Wesentlichen in axialer Richtung des Außengewindes verlaufende Rippe zwischen zwei benachbarten Gewindeflanken des Außengewindes im Inneren der Mutter gegen die Gewindeflanke des Innengewindes der Mutter drückt und/oder auf der Gewindeflanke entlanggleitet.

Anders ausgedrückt trifft die erste Ausformung in dem vorgegebenen ersten Drehwinkelbereich auf keine zweite Ausformung, so dass die erste Ausformung in dem vorgegebenen ersten Drehwinkelbereich in keine zweite Ausformung einrasten kann.

Das Innengewinde und das Außengewinde sowie die erste Ausformung und die zweite Ausformung sind des Weiteren konfiguriert zum Einrasten der ersten Ausformung in die zweite Ausformung, wenn das Innengewinde und das Außengewinde in einem vorgegebenen zweiten Drehwinkelbereich verschraubt sind.

Beispielsweise rastet in einer Ausführungsform eine im Wesentlichen in axialer Richtung des Außengewindes verlaufende Rippe zwischen zwei benachbarten Gewindeflanken des Außengewindes in eine im Wesentlichen in axialer Richtung des Innengewindes verlaufende Nut in der Gewindeflanke des Innengewindes ein, wenn das Innengewinde und das Außengewinde in dem vorgegebenen zweiten Drehwinkelbereich verschraubt sind.

Der zweite Drehwinkelbereich kann durch die Form und die Anordnung der ersten und/oder der zweiten Ausformung festgelegt sein. Der zweite Winkelbereich kann in manchen Ausführungsformen im Wesentlichen durch die Breite der ersten Ausformung und die Breite der zweiten Ausformung in Drehrichtung des Gewindes festgelegt sein. Dabei kann die zweite Ausformung gegenüber der ersten Ausformung ein Übermaß aufweisen, so dass sich ein Spiel ergibt. Das Spiel kann das Einrasten der ersten Ausformung in die zweite Ausformung erleichtern.

In einer weiteren Ausführungsform kann das zweite Verbindungselement eine Abdichtkappe mit einer Abdichtstruktur aufweisen oder eine Abdichtkappe mit einer Abdichtstruktur sein. Eine solche Ausführungsform kann beispielsweise vorliegen, wenn das zweite Verbindungselement als Berührschutzkappe und/oder Verschlusskappe konfiguriert ist. Die Abdichtstruktur kann zum gasdichten und/oder flüssigkeitsdichten Abdichten mit einem ersten Verbindungselement konfiguriert sein.

In einer weiteren Ausführungsform weist das zweite Verbindungselement ein als zweites Lumen bezeichnetes Lumen auf und die Mutter ist als Überwurfmutter konfiguriert. Die Überwurfmutter ist drehbar am zweiten Verbindungselement gelagert, so dass die Überwurfmutter mit einem ersten Verbindungselement verschraubt werden kann, ohne dass sich das zweite Verbindungselement verdreht. Eine solche Ausführungsform ist besonders vorteilhaft, wenn der erfindungsgemäße Schraubverbinder zum Verbinden von zwei Schlauchleitungsabschnitten vorgesehen ist, die sich beim Verschrauben des Schraubverbinders nicht verdrehen sollen.

Weiter vorteilhaft wird ein Schraubverbinder für medizinische Schlauchsysteme bereitgestellt, der eine halboffene Sterilisierstellung zulässt und zugleich ergonomisch und sicher in der Handhabung ist. Gleichzeitig ist die halboffene Sterilisierstellung verlässlich und sicher.

In einer weiteren Ausführungsform ist der Schraubverbinder konfiguriert um in einem weiteren Drehwinkelbereich gas- und/oder flüssigkeitsdicht verschraubt zu werden. Dabei sind das erste Verbindungselement und das zweite Verbindungselement jeweils konfiguriert um beim Verschrauben des Innengewindes mit dem Außengewinde in dem zweiten Drehwinkelbereich zwischen einer äußeren Umgebung des Schraubverbinders und einem zumindest durch das erste Lumen gebildeten Inneren des Schraubverbinders gasdurchlässig zu sein und außerdem sind das erste Verbindungselement und das zweite Verbindungselement jeweils konfiguriert um beim Verschrauben des Innengewindes mit dem Außengewinde in dem weiteren Drehwinkelbereich gasdicht und/oder flüssigkeitsdicht zwischen der äußeren Umgebung des Schraubverbinders und dem Inneren des Schraubverbinders abzudichten. Zum Verschrauben in dem weiteren Drehwinkelbereich ist zunächst ein Überschreiten des zweiten Drehwinkelbereichs erforderlich. Anders ausgedrückt wird der Schraubverbinder über den zweiten Drehwinkelbereich hinaus weiter zugedreht. Dabei rastet zunächst die erste Ausformung aus der zweiten Ausformung aus. Für das Ausrasten der ersten Ausformung aus der zweiten Ausformung ist ein Drehmoment an der Mutter erforderlich. Die gasdichte und/oder flüssigkeitsdichte Stellung wird erst durch vollständiges Zudrehen der Schraubverbindung erreicht.

Im Einklang mit der Lehre der vorliegenden Erfindung können das erste Verbindungselement und das zweite Verbindungselement jeweils konfiguriert sein, um beim Verschrauben des Innengewindes mit dem Außengewinde in dem mindestens einen vorgegebenen zweiten Drehwinkelbereich eine halboffene Sterilisierstellung des Schraubverbinders für eine Gas- und/oder Dampfsterilisierung des medizinischen Schlauchsystems einzustellen. Die halboffene Sterilisierstellung wird eingestellt, indem die Stellung des Innengewindes zum Außengewinde in dem zweiten Drehwinkelbereich fixiert ist, wobei bei dieser Stellung zwischen dem ersten Lumen und dem zweiten Lumen ein Spalt besteht, so dass Gas- und/oder Dampf in des Innere des ersten Lumens und/oder des zweiten Lumens eindringen kann. Die Fixierung wird erreicht, weil in dem mindestens einen vorgegebenen zweiten Drehwinkelbereich die erste Ausformung in die zweite Ausformung einrastet. Somit kann eine unbeabsichtigte Veränderung der halboffenen Sterilisierstellung sicher verhindert werden.

In einer weiteren Ausführungsform ist der Schraubverbinder Teil eines medizinischen Schlauchsystems. Der Begriff "medizinisches Schlauchsystem" umfasst in der vorliegenden Erfindung alle medizinischen Produkte, die zumindest einen Schlauchabschnitt aufweisen und insbesondere für die extrakorporale Blutbehandlung, insbesondere für die Hämodialyse, Hämofiltration oder Hämodiafiltration, oder für die Peritonealdialyse oder für die Infusion von medizinischen Lösungen in den Patienten konfiguriert und/oder vorgesehen sind.

Eine Ausführungsform des erfindungsgemäßen medizinischen Schlauchsystems ist ein Blutschlauchsatz für die extrakorporale Blutbehandlung mit einem arteriellen Patientenschlauch und einem venösen Patientenschlauch, wobei zumindest das patientenseitige Ende des venösen Patientenschlauchs und/oder das patientenseitige Ende des arteriellen Patientenschlauchs als das erste Verbindungselement oder das zweite Verbindungselement des erfindungsgemäßen Schraubverbinders ausgebildet sind.

In einer Ausführungsform wird ein vor-konnektierter Blutschlauchschlauchsatz für die extrakorporale Blutbehandlung mit einem Drei-Wege-Verbinder bereitgestellt, der zum Sterilisieren des Blutschlauchsatzes eine halboffene Sterilisierstellung der an dem Drei-Wege-Verbinder vor-konnektierten Luer-Lock-Verbindungen des arteriellen und des venösen Patientenkonnektors ermöglicht. Der Drei-Wege-Verbinder weist drei Anschlüsse zu drei verbundenen Lumen auf. Dabei ist zumindest ein Anschluss des Drei-Wege-Verbinders als erstes Verbindungselement, insbesondere als weibliche Hälfte des Luer-Lock Verbinders ausgestaltet und zumindest das patientenseitige Ende des venösen Patientenschlauchs und/oder das patientenseitige Ende des arteriellen Patientenschlauchs als das zweite Verbindungselement des erfindungsgemäßen Schraubverbinders, insbesondere als männliche Hälfte des Luer-Lock Verbinders ausgebildet. Besonders bevorzugt sind zumindest zwei Anschlüsse des Drei-Wege-Verbinders als das erste Verbindungselement, insbesondere als weibliche Hälfte eines Luer-Lock Verbinders ausgestaltet und sowohl das patientenseitige Ende des venösen Patientenschlauchs als auch das patientenseitige Ende des arteriellen Patientenschlauchs als das zweite Verbindungselement des erfindungsgemäßen Schraubverbinders, insbesondere als männliche Hälfte des Luer-Lock Verbinders ausgebildet. Dabei ergibt sich der zusätzliche Vorteil, dass zumindest zwei Verschlusskappen zum Verschließen des patientenseitigen Endes des venösen Patientenschlauchs und des patientenseitigen Endes des arteriellen Patientenschlauchs eingespart werden, weil diese Funktion durch den Drei-Wege-Verbinder erfüllt wird. Der dritte Anschluss des Drei-Wege-Verbinders kann mit einer konventionellen Abdeckkappe verschlossen sein oder aber ebenfalls mit einem Schraubverbinder ausgestattet sein, wobei der Schraubverbinder dann bevorzugt als Abschlusskappe ausgeführt ist. Das medizinische Schlauchsystem ist somit konfiguriert zum Kurzschließen des patientenseitigen Endes des arteriellen Patientenschlauchs und des patientenseitigen Endes des venösen Patientenschlauchs mit dem Drei-Wege-Verbinder. Dies hat den Vorteil, dass das medizinische Schlauchsystem nach dem vollständigen Zudrehen der Luer-Lock Verbinder mittels des dritten Anschlusses mit einer Quelle für medizinische Primingflüssigkeit verbunden und mit der medizinischen Primingflüssigkeit gefüllt und gespült werden kann. Das medizinische Schlauchsystem ist somit vor-konnektiert zum Zwecke des Spülens mit medizinischer Primingflüssigkeit und somit praktisch in der Handhabung.

Somit wird vorteilhaft ein vor-konnektierter Blutschlauchschlauchsatz für die extrakorporale Blutbehandlung mit einem Drei-Wege-Verbinder bereitgestellt, der zum Sterilisieren des Blutschlauchsatzes eine halboffene Sterilisierstellung der an dem Drei-Wege-Verbinder vor-konnektierten Luer-Lock-Verbindungen des arteriellen und des venösen Patientenkonnektors ermöglicht und zugleich ergonomisch und sicher in der Handhabung ist. Dabei ergibt sich der zusätzliche Vorteil, dass zumindest zwei Verschlusskappen zum Verschließen des arteriellen und des venösen Patientenkonnektors eingespart werden.

In einer Ausführungsform weist das medizinische Schlauchsystem eine Blutkassette für die extrakorporale Blutbehandlung auf, wobei das dem patientenseitigen Ende des arteriellen Patientenschlauchs gegenüber liegende Ende des arteriellen Patientenschlauchs und das dem patientenseitigen Ende des venösen Patientenschlauchs gegenüber liegende Ende des venösen Patientenschlauchs jeweils mit der Blutkassette für die extrakorporale Blutbehandlung verbunden sind. Die Blutkassette kann weitere Elemente eines extrakorporalen Blutkreislaufs beinhalten, beispielsweise ausgewählt aus Luftblasenabscheider, Gerinnselfilter, Ports, Kavitäten und Leitungen zum Aufnehmen und/oder Führen von Blut und/oder medizinischen Flüssigkeiten sowie Ventile und Pumpelemente. Bei einer solchen Ausführungsform können auch Ports der Blutkassette und weitere Schlauchleitungsabschnitte an der Blutkassette mit einem erfindungsgemäßen Schraubverbinder ausgestattet sein, wobei der Schraubverbinder dann bevorzugt als Abschlusskappe ausgebildet ist.

In einer Ausführungsform ist das medizinische Schlauchsystem in einer geschlossenen Umhüllung steril verpackt, wobei das patientenseitige Ende des arteriellen Patientenschlauchs und das patientenseitige Ende des venösen Patientenschlauchs in der halboffenen Sterilisierstellung mit einem Drei-Wege-Verbinder verbunden sind.

Im Einklang mit der Lehre der vorliegenden Erfindung ist der Schraubverbinder zur Verwendung zum Gassterilisieren und/oder Dampfsterilisieren eines medizinischen Schlauchsystems geeignet und vorgesehen.

Die Erfinder der vorliegenden Erfindung haben erkannt, dass die komplementären Hälften eines vom Standard abweichenden Schraubverbinders eines medizinischen Schlauchsatzes beim Einsatz in der Klinik nicht nur eingeschränkt, sondern möglichst flexibel, insbesondere kompatibel einsetzbar sein sollten.

In einer bestimmten Ausführungsform des Schraubverbinders ist das zweite Verbindungselement mit einem Gegenstück eines standardisierten Luer-Lock Verbinders zum flüssigkeitsdichten Verschrauben kompatibel, weil die zweite Ausformung dabei nicht störend wirken kann. Die Verwendung des erfindungsgemäßen Schraubverbinders beim Sterilisieren eines medizinischen Schlauchsatzes schränkt daher die Verwendung des medizinischen Schlauchsatzes in der Klinik kaum ein. Daher ist das zweite Verbindungselement kompatibel und daher flexibel im Klinikbetrieb einsetzbar. Als ein Beispiel werden Dialysenadeln angeführt, die einen kurzen Schlauchabschnitt mit einer weiblichen Hälfte eines genormten Luer-Lock Verbinders aufweisen. Diese sind ohne Einschränkung mit dem zweiten Verbindungselement eines erfindungsgemäßen Schraubverbinders verbindbar und zum Verbinden vorgesehen. Daher kann das zweite Verbindungselement des erfindungsgemäßen Schraubverbinders, dass an dem patientenseitigen Ende des venösen Patientenschlauchs und dem patientenseitigen Ende des arteriellen Patientenschlauchs vorgesehen sein kann, jeweils mit der eine weibliche Hälfte eines genormten Luer-Lock Verbinders aufweisenden Dialysenadel verbunden werden.

Der Schraubverbinder ist vorzugsweise unempfindlich gegenüber axialem Druck oder Zug am ersten und/oder zweiten Verbindungselement wenn das Innengewinde mit dem Außengewinde verschraubt ist. Der Schraubverbinder ist dann robust gegenüber dem unbeabsichtigten Diskonnektieren aufgrund von Zug- und/oder Biegebeanspruchungen der Verbindungsteile, wie diese bei Verpackung, Lagerung und Transport auftreten können, weil die Gewindeflanken die Zug- und Biegekräfte aufnehmen. Das Lösen ist nur durch eine gezielte Drehbewegung über einen Widerstand hinaus möglich.

Die elastische Verformung der Mutter oder Überwurfmutter beim Verschrauben in dem ersten Drehwinkelbereich beträgt vorzugsweise 0,15 bis 0,25 Millimeter, insbesondere bei standardisierten verriegelbaren Luerverbindungen und entspricht dem Übermaß zwischen der ersten Ausformung und dem Innengewinde

Der Schraubverbinder kann in einer Ausführungsform für nur ein einmaliges Einrasten der ersten Ausformung in die zweite Ausformung konfiguriert sein. Ein mehrmaliges Einrasten ist in solchen Ausführungsformen nicht vorgesehen, weil die erste Ausformung und/oder die zweite Ausformung beim Einrasten und Verschrauben über die Einrastung hinaus verschleißen. Solche Ausführungsformen sind aufgrund der geringen Anforderungen an Haltbarkeit der ersten Ausformung und/oder der zweite Ausformung besonders kostengünstig.

In einer Ausführungsform des Schraubverbinders ist eine Führungshilfe beziehungsweise Führungsstrecke vorgesehen, die die Handhabung durch den Anwender in der Klinik erleichtert und außerdem eine Montage durch Roboter ermöglicht. Die Führungshilfe kann in manchen Ausführungsformen durch das Gewinde selbst realisiert sein.

Somit wird vorteilhaft ein Schraubverbinder bereitgestellt, der ergonomisch und sicher in der Handhabung ist.

Die Hälften des Schraubverbinders können so beim manuellen Zuschrauben und Abschrauben in der Klinik sicher geführt und gehalten werden, was besonders vorteilhaft ist, wenn der Schraubverbinder kleinteilig ist. Der Außendurchmesser der Mutter des erfindungsgemäßen Schraubverbinders kann beispielsweise 6 Millimeter bis 12 Millimeter oder auch 12 Millimeter bis 25 Millimeter betragen.

Typischerweise erfolgt das manuelle Auf- und/oder Zuschrauben des Schraubverbinders durch Verdrehen der Mutter oder der Überwurfmutter des zweiten Verbindungselements zwischen den Fingerkuppen von Daumen und Zeigefinger der einen Hand des Anwenders während das erste Verbindungselement von den Fingern der anderen Hand festgehalten wird. Beim Auf- und/oder Zuschrauben des Schraubverbinders spürt der Anwender zum einen von Anfang an, dass das erste Verbindungselement gegenüber dem zweiten Verbindungselement um einen Drehwinkel verdreht und vom Gewinde sicher geführt wird. Zum anderen spürt der Anwender in Abhängigkeit vom Drehwinkel einen veränderten Widerstand beim Verdrehen, beispielsweise erstens wenn die Mutter oder Überwurfmutter unter deren elastischer Verformung durch die erste Ausformung in dem vorgegebenen ersten Drehwinkelbereich verdreht wird, ohne dass die erste Ausformung in die zweite Ausformung einrastet und zweitens wenn die erste Ausformung in die zweite Ausformung einrastet, wenn das Innengewinde und das Außengewinde in einem vorgegebenen zweiten Drehwinkelbereich verschraubt sind. Des Weiteren kann der Anwender einen veränderten Widerstand beim Verdrehen spüren, wenn das Innengewinde und das Außengewinde über den vorgegebenen zweiten Drehwinkelbereich hinaus verschraubt und flüssigkeitsdicht zugeschraubt werden. Der Anwender kann diese veränderlichen Widerstände jeweils auch beim Aufschrauben bis zum Trennen des ersten Verbindungselements von dem zweiten Verbindungselement spüren. Wenn der Schraubverbinder mehrere Einraststellungen aufweist, dann kann der Anwender die veränderlichen Widerstände bei jeder Einraststellung spüren.

Der Schraubverbinder ermöglicht somit durch den drehwinkelabhängigen Drehwiderstand ein taktiles Feedback bei der manuellen Handhabung des erfindungsgemäßen Schraubverbinders, das heißt die Vermittlung des Gefühls der Ertastbarkeit des Handhabungsfortschritts mit den Fingern. Vorteilhaft ist dabei der durch das Gewinde verlängerte Weg zwischen dem nicht verrasteten Zustand und dem verrasteten Zustand und umgekehrt.

So wird ein Schraubverbinder bereitgestellt, der vorteilhaft auch ergonomisch und sicher in der Handhabung ist.

Die Mutter oder die Überwurfmutter des zweiten Verbindungselements kann durch die Auswahl eines geeigneten elastischen Werkstoffs und die Formgebung zur elastischen Verformung durch die erste Ausformung des ersten Verbindungselements konfiguriert sein.

Als Werkstoff für das erste und das zweite Verbindungselement eignen sich beispielsweise Kunststoffe.

Als Werkstoff für das erste und das zweite Verbindungselement eignet sich in manchen Ausführungsformen insbesondere PP (Polypropylen).

### Kurze Beschreibung der Zeichnungen

Im Folgenden wird ein Ausführungsbeispiel nach der vorliegenden Lehre unter Bezugnahme auf die Figuren näher erläutert. Anhand des in den Figuren dargestellten Ausführungsbeispiels werden weitere Einzelheiten und Vorteile näher beschrieben. Die Bezugszeichen in den Figuren haben jeweils durchgängig in allen Figuren die gleiche Bedeutung.

Es zeigen:
**Fig. 1** eine Ansicht eines Abschnitts eines medizinischen Blutschlauchsatzes
**Fig. 2** eine Schnittdarstellung des Abschnitts eines medizinischen Blutschlauchsatzes gemäß Figur 1
**Fig. 3** eine Detaildarstellung X aus dem Abschnitt eines medizinischen Blutschlauchsatzes gemäß Figur 1.

### Detaillierte Beschreibung der Zeichnungen

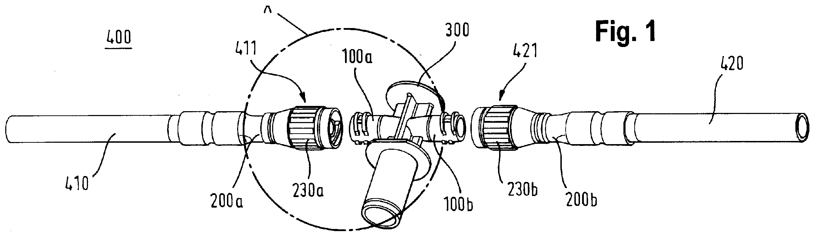

Die **Figur 1** zeigt eine Ansicht eines Abschnitts eines medizinischen Schlauchsystems, bevorzugt eines medizinischen Blutschlauchsatzes mit einer arteriellen Patientenleitung (410) mit einem patientenseitigen Ende (411) sowie einer venösen Patientenleitung (420) mit einem patientenseitigen Ende (421) sowie einem Drei-Wege-Verbinder (300), wobei die arterielle Patientenleitung (410) und die venöse Patientenleitung (420) nicht mit dem Drei-Wege-Verbinder (300) verbunden sind. Der Drei-Wege-Verbinder (300) weist zwei Anschlüsse auf, die als erstes Verbindungselement (100a, 100b) eines Schraubverbinders ausgebildet sind. Bevorzugt ist das erste Verbindungselement (100a, 100b) jeweils als weibliche Hälfte eines Luer-Lock Verbinders ausgebildet. Das patientenseitige Ende (421) der venösen Patientenleitung (420) und das patientenseitige Ende (411) der arteriellen Patientenleitung (410) sind jeweils als zweites Verbindungselement (200) des Schraubverbinders ausgebildet. Bevorzugt ist das zweite Verbindungselement (200a, 200b) jeweils als männliche Hälfte des Luer-Lock Verbinders ausgebildet und weist jeweils eine Überwurfmutter (230a, 230b) auf, die zum Verschrauben mit der weiblichen Hälfte des Luer-Lock Verbinders ausgebildet ist.

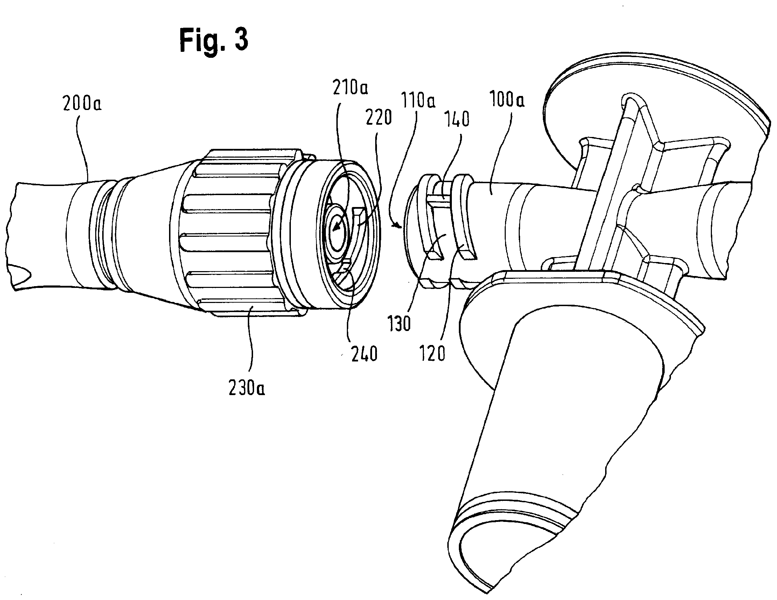

Die **Figur 1** zeigt des Weiteren einen mittels einer strichpunktierten Linie gekennzeichneten Bereich X, der als vergrößertes Detail in **Figur 3** gezeigt wird.

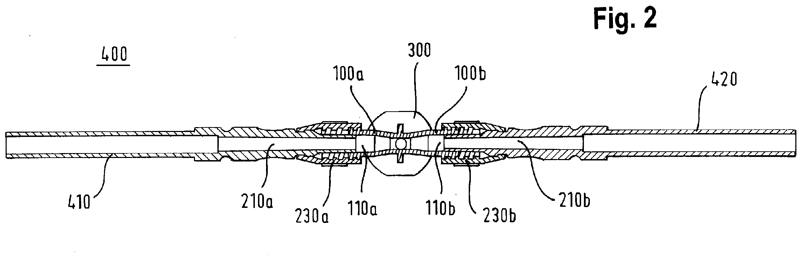

Die **Figur 2** zeigt eine Schnittdarstellung des Abschnitts eines medizinischen Blutschlauchsatzes aus **Figur 1**, wobei in **Figur 2** die arterielle Patientenleitung (410) und die venöse Patientenleitung (420) mit dem Drei-Wege-Verbinder (300) verbunden sind. In der Schnittdarstellung ist zu erkennen, dass der Drei-Wege-Verbinder (300) erste Verbindungselemente (100a, 100b) sowie erste Lumen (110a, 110b) aufweist. Bevorzugt sind die ersten Verbindungselemente (100a, 100b) jeweils als weibliche Hälfte des Luer-Lock Verbinders ausgebildet.

Das patientenseitigen Ende (421) der venösen Patientenleitung (420) und das patientenseitigen Ende (411) der arteriellen Patientenleitung (410) sind jeweils als zweites Verbindungselement (200a, 200b) mit einem zweiten Lumen (210a, 210b) ausgebildet. Bevorzugt ist das zweite Verbindungselement (200a, 200b) jeweils als männliche Hälfte des Luer-Lock Verbinders ausgebildet und weist jeweils eine Überwurfmutter (230a, 230b) auf, die jeweils mit der komplementären weiblichen Hälfte des Luer-Lock Verbinder verschraubt ist.

Das Innengewinde und das Außengewinde sind jeweils als komplementäre Gewinde des Luer-Lock Verbinders ausgeführt. Die komplementären Gewinde des Luer-Lock Verbinders sind zumindest hinsichtlich Gewindeform, Gewindedurchmesser, Gewindesteigung und Gewindelänge kompatibel ausgeführt und so zum gegenseitigen Verschrauben konfiguriert und vorgesehen.

Die **Figur 3** zeigt die Detaildarstellung X der Ansicht aus dem Abschnitt eines medizinischen Blutschlauchsatzes gemäß **Figur 1****.** Die Beschreibung des Schraubverbinders erfolgt beispielhaft für den in der Detaildarstellung X gezeigten linken Schraubverbinder (200a, 230a, 100a) aus **Figur 1**, gilt aber gleichermaßen auch für den nicht als Detail gezeigten rechten Schraubverbinder ((200b, 230b, 100b) aus **Figur 1****.**

Die Detaildarstellung X zeigt vergrößert das patientenseitige Ende (411) der arteriellen Leitung (410) mit dem zweiten Verbindungselement (200a), das die Überwurfmutter (230a) aufweist. Die Detaildarstellung zeigt im Bereich des offenen Endes der Überwurfmutter (230a) die Mündung des zweiten Lumens (210a) und einen Abschnitt des Innengewindes (220). Das Innengewinde (220) weist die zweite Ausformung (240) auf. Bevorzugt ist die zweite Ausformung (240) als im Wesentlichen in axialer Richtung des Innengewindes verlaufende Nut in der Gewindeflanke des Innengewindes (220) ausgebildet. Das erste Verbindungselement (100a) mit dem Lumen (110a) weist das Außengewinde (120) mit dem Gewindegrund (130) und der ersten Ausformung (140) auf. Bevorzugt ist die erste Ausformung (140) als eine im Wesentlichen in axialer Richtung des Außengewindes verlaufende Rippe zwischen zwei benachbarten Gewindeflanken des Außengewindes ausgestaltet. Die im Wesentlichen in axialer Richtung des Außengewindes verlaufende Rippe stellt somit eine lokale Erhebung auf dem Gewindegrund (130) des Außengewindes (120) dar.

Das zweite Lumen (210a) ist ausgebildet, um in das erste Lumen (110a) einzugreifen, wenn der Luer-Lock Verbinder verschraubt ist. Daher ist das zweite Verbindungselement (200a) als männliche Hälfte des Luer-Lock Verbinders ausgebildet und das erste Verbindungselement (100a) als weibliche Hälfte des Luer-Lock Verbinders ausgebildet.

Die Überwurfmutter (230) ist bevorzugt aus Kunststoff, insbesondere Polypropylen (PP) hergestellt und daher konfiguriert zur elastischen Verformung durch die erste Ausformung (140) in einem vorgegebenen ersten Drehwinkelbereich, in dem die erste Ausformung (140) nicht in die zweite Ausformung (240) einrastet. Dazu kann der Durchmesser der Überwurfmutter (230) beim Verdrehen in einem ersten Drehwinkelbereich elastisch aufgeweitet werden, wenn die im Wesentlichen in axialer Richtung des Außengewindes verlaufende Rippe zwischen zwei benachbarten Gewindeflanken des Außengewindes (120) im Inneren der Mutter gegen die Gewindeflanke des Innengewindes (220) der Überwurfmutter (230) drückt und/oder auf der Gewindeflanke des Innengewindes (220) entlanggleitet. Daher trifft die erste Ausformung (140) in dem vorgegebenen ersten Drehwinkelbereich nicht auf die zweite Ausformung (240), so dass die erste Ausformung (140) in dem vorgegebenen ersten Drehwinkelbereich nicht in die zweite Ausformung (240) einrasten kann. Das Innengewinde (220) und das Außengewinde (120) sowie die erste Ausformung (140) und die zweite Ausformung (240) sind konfiguriert zum Einrasten der ersten Ausformung (140) in die zweite Ausformung (240) wenn das Innengewinde (220) und das Außengewinde (120) in einem vorgegebenen zweiten Drehwinkelbereich verschraubt sind. Der Verschraubungszustand bei dem zweiten Drehwinkelbereich stellt die halboffene Sterilisierstellung des Schraubverbinders für eine Gas- und/oder Dampfsterilisierung des medizinischen Schlauchsystems ein. Die halboffene Sterilisierstellung wird eingestellt, indem die Stellung des Innengewindes (220) zum Außengewinde (120) in dem zweiten Drehwinkelbereich fixiert ist, wobei bei dieser Stellung zwischen dem ersten Lumen und dem zweiten Lumen ein Spalt besteht, so dass Gas- und/oder Dampf in des Innere des ersten Lumens und des zweiten Lumens eindringen kann. Die Fixierung wird erreicht, indem in dem mindestens einen vorgegebenen zweiten Drehwinkelbereich die erste Ausformung (140) in die zweite Ausformung (240) einrastet. Somit wird eine unbeabsichtigte Veränderung der halboffenen Sterilisierstellung sicher verhindert, so dass der Spalt verlässlich eingestellt bleibt.

Der Schraubverbinder ist ausgebildet um in einem weiteren Drehwinkelbereich gas- und/oder flüssigkeitsdicht verschraubt zu werden. Zum Verschrauben in dem dritten Drehwinkelbereich ist zunächst ein Überschreiten des zweiten Drehwinkelbereichs erforderlich, wobei der Schraubverbinder über den zweiten Drehwinkelbereich hinaus weiter zugedreht wird. Dabei rastet zunächst die erste Ausformung (140) aus der zweiten Ausformung (240) aus. Für das Ausrasten der ersten Ausformung (140) aus der zweiten Ausformung (240) ist ein Drehmoment an der Mutter erforderlich. Die gas- und/oder flüssigkeitsdichte Stellung wird erst durch vollständiges Zudrehen der Schraubverbindung erreicht. Vorteilhaft erfolgt das vollständige Zuschrauben des Schraubverbinders manuell nach dem Entnehmen des sterilen medizinischen Schlauchsystems aus der geschlossenen sterilen Umhüllung. Sobald der medizinische Schraubverbinder gas- und flüssigkeitsdicht zugeschraubt ist, kann der dritte Anschluss des Drei-Wege-Verbinders an einer Quelle für eine medizinische Primingflüssgkeit angeschlossen werden, um das medizinische Schlauchsystem mit medizinischer Primingflüssgkeit zu befüllen und zu spülen.

Das medizinische Schlauchsystem weist gemäß dem Ausführungsbeispiel eine Blutkassette für die extrakorporale Blutbehandlung auf, wobei das dem patientenseitigen Ende des arteriellen Patientenschlauchs gegenüber liegende Ende des arteriellen Patientenschlauchs und das dem patientenseitigen Ende des venösen Patientenschlauchs gegenüber liegende Ende des venösen Patientenschlauchs jeweils mit der Blutkassette für die extrakorporale Blutbehandlung verbunden ist (nicht in den Figuren 1 bis 3 dargestellt).

### Bezugszeichenliste

**Tabelle 1**

| **Bezugszeichen** | **Bezeichnung** |
|---|---|
| 100, 100a, 100b | Erstes Verbindungselement |
| 110, 110a, 110b | Erstes Lumen |
| 120 | Außengewinde |
| 130 | Gewindegrund |
| 140 | Erste Ausformung |
| 200, 200a, 200b | Zweites Verbindungselement |
| 210, 210a, 210b | Zweites Lumen |
| 220 | Innengewinde |
| 230, 230a, 230b | Überwurfmutter |
| 240 | Zweite Ausformung |
| 300 | Drei-Wege-Verbinder |
| 400 | Medizinischer Schlauchsatz |
| 410 | Arterielle Patientenleitung |
| 411 | patientenseitiges Ende der arterielle Patientenleitung |
| 420 | Venöse Patientenleitung |
| 421 | patientenseitiges Ende der venösen Patientenleitung |

## Patentansprüche

1. Schraubverbinder für ein medizinisches Schlauchsystem mit mindestens einem ersten Verbindungselement (100, 100a, 100b) mit einem ersten Lumen (110, 110a, 110b) und einem Außengewinde (120) und mindestens einem zweiten Verbindungselement (200, 200a, 200b) mit einem zweiten Lumen (210, 210a, 210b) und einer elastisch verformbaren Überwurfmutter (230, 230a, 230b) mit einem Innengewinde (220), wobei das Innengewinde (220) und das Außengewinde (120) konfiguriert sind um miteinander verschraubt zu werden wobei
das Außengewinde mindestens eine erste Ausformung (140) aufweist und das Innengewinde mindestens eine zweite Ausformung (240) aufweist und
das Innengewinde (220) und das Außengewinde (120) konfiguriert sind zum gegenseitigen Verschrauben unter elastischer Verformung der Überwurfmutter (230, 230a, 230b) mittels der ersten Ausformung (140) in mindestens einem vorgegebenen ersten Drehwinkelbereich, ohne dass die erste Ausformung (140) in die zweite Ausformung (240) einrastet und das Innengewinde (220) und das Außengewinde (120) und die erste Ausformung (140) und die zweite Ausformung (240) konfiguriert sind zum Einrasten der ersten Ausformung (140) in die zweite Ausformung (240) wenn das Innengewinde (220) und das Außengewinde (120) in einem vorgegebenen zweiten Drehwinkelbereich verschraubt sind.
**dadurch gekennzeichnet, dass**
das erste Verbindungselement (100, 100a, 100b) und das zweite Verbindungselement (200, 200a, 200b) jeweils
konfiguriert sind um beim Verschrauben des Innengewindes (220) mit dem Außengewinde (120) in dem mindestens einen vorgegebenen zweiten Drehwinkelbereich gasdurchlässig zu sein zwischen einer äußeren Umgebung des Schraubverbinders und einem durch das erste Lumen (110, 110a, 110b) und das zweite Lumen (210, 210a, 210b) gebildeten Inneren des Schraubverbinders und
das erste Verbindungselement (100, 100a, 100b) und das zweite Verbindungselement (200, 200a, 200b) jeweils konfiguriert sind um beim Verschrauben des Innengewindes (220) mit dem Außengewinde (120) in einem weiteren Drehwinkelbereich gas- und/oder flüssigkeitsdicht zwischen der äußeren Umgebung des Schraubverbinders und dem Inneren des Schraubverbinders abzudichten.

2. Schraubverbinder gemäß Anspruch 1, wobei das erste Verbindungselement (100, 100a, 100b) und das zweite Verbindungselement (200, 200a, 200b) jeweils als komplementäre Hälfte eines gemeinsamen Luer-Verbinders ausgeführt sind

3. Schraubverbinder gemäß Anspruch 2, wobei das erste Lumen (110, 110a, 110b) als weibliche Hälfte des Luer-Konnektors ausgebildet ist und das zweite Lumen (210, 210a, 210b) als männliche Hälfte des Luer-Konnektors ausgebildet ist.

4. Schraubverbinder gemäß einem der Ansprüche 1 bis 3, wobei die erste Ausformung (140) eine lokale Erhebung auf dem Kerndurchmesser des Außengewindes (120) zwischen zwei benachbarten Gewindeflanken ist und die zweite Ausformung (240) eine lokale Freistellung an der Gewindeflanke des Innengewindes (220) ist.

5. Schraubverbinder gemäß Anspruch 4, wobei die lokale Freistellung als eine im Wesentlichen in axialer Richtung des Innengewindes (220) verlaufende Nut in einer Gewindeflanke des Innengewindes (220) ausgestaltet ist und die lokale Erhebung als eine im Wesentlichen in axialer Richtung des Außengewindes (120) verlaufende Rippe zwischen zwei benachbarten Gewindeflanken des Außengewindes (120) ausgestaltet ist.

6. Schraubverbinder gemäß Anspruch 1, wobei das erste Verbindungselement (100, 100a, 100b) und das zweite Verbindungselement (200, 200a, 200b) jeweils konfiguriert sind um beim Verschrauben des Innengewindes (220) mit dem Außengewinde (120) in dem mindestens einen vorgegebenen zweiten Drehwinkelbereich eine halboffene Sterilisierstellung des Schraubverbinders für eine Gas- und/oder Dampfsterilisierung des medizinischen Schlauchsystems einzustellen.

7. Medizinisches Schlauchsystem mit einem Schraubverbinder gemäß einem der Ansprüche 1 bis 6, wobei das medizinische Schlauchsystem einen Blutschlauchsatz für die extrakorporale Blutbehandlung mit einem arteriellen Patientenschlauch und einem venösen Patientenschlauch aufweist und das patientenseitige Ende des arteriellen Patientenschlauchs (411) und das patientenseitige Ende des venösen Patientenschlauchs (421) jeweils als ein zweites Verbindungselement (200, 200a, 200b) ausgebildet sind.

8. Medizinisches Schlauchsystem gemäß Anspruch 7, wobei das medizinische Schlauchsystem einen Drei-Wege-Verbinder (300) mit drei verbundenen Lumen aufweist, wobei zumindest zwei der drei verbundenen Lumen jeweils als das erste Verbindungselement (100, 100a, 100b) ausgestaltet sind.

9. Medizinisches Schlauchsystem gemäß Anspruch 8 konfiguriert zum Kurzschließen des patientenseitigen Endes des arteriellen Patientenschlauchs (411) und des patientenseitige Ende des venösen Patientenschlauchs (421) mit dem Drei-Wege-Verbinder (300).

10. Medizinisches Schlauchsystem gemäß Anspruch 8, wobei das dem patientenseitigen Ende des arteriellen Patientenschlauchs (411) gegenüber liegende Ende des arteriellen Patientenschlauchs und das dem patientenseitigen Ende des venösen Patientenschlauchs (421) gegenüber liegende Ende des venösen Patientenschlauchs mit einer Blutkassette für die extrakorporale Blutbehandlung verbunden sind.

11. Medizinisches Schlauchsystem gemäß Anspruch 10, wobei das medizinische Schlauchsystem in einer geschlossenen Umhüllung steril verpackt ist und das patientenseitige Ende des arteriellen Patientenschlauchs (411) und das patientenseitige Ende des venösen Patientenschlauchs (421) in der halboffenen Sterilisierstellung mit dem Drei-Wege-Verbinder (300) verbunden sind.

12. Verwenden eines Schraubverbinders gemäß einem der Ansprüche 1 bis 6 zum Gassterilisieren und/oder Dampfsterilisieren eines medizinischen Schlauchsystems gemäß einem der Ansprüche 7 bis 11.

## Claims

1. A screw connector for a medical hose system, comprising at least one first connecting element (100, 100a, 100b) having a first lumen (110, 110a, 110b) and an outside thread (120) and at least one second connecting element (200, 200a, 200b) having a second lumen (210, 210a, 210b) and an elastically deformable union nut (230, 230a, 230b) having an inside thread (220), wherein the inside thread (220) and the outside thread (120) are configured to be screwed to one another, wherein the outside thread has at least one first formation (140) and the inside thread has at least one second formation (240), and the inside thread (220) and the outside thread (120) are configured for mutual screw connection with elastic deformation of the union nut (230, 230a, 230b) by means of the first formation (140) in at least one predetermined first angle-of-rotation range without the first formation (140) engaging in the second formation (240), and the inside thread (220) and the outside thread (120) and the first formation (140) and the second formation (240) are configured for catch engagement of the first formation (140) in the second formation (240) when the inside thread (220) and the outside thread (120) are screwed together in a predefined second angle-of-rotation range, **characterized in that**
the first connecting element (100, 100a, 100b) and the second connecting element (200, 200a, 200b) are each configured to be gas-permeable between an outer environment of the screw connector and an interior of the screw connector formed by the first lumen (110, 110a, 110b) and the second lumen (210, 210a, 210b) in the at least one predetermined second angle-of-rotation range in screw connection of the inside thread (220) with the outside thread (120), and the first connecting element (100, 100a, 100b) and second connecting element (200, 200a, 200b) are each configured in order to seal in a gas-tight and/or fluid-tight manner between the outer environment of the screw connector and the interior of the screw connector when the inside thread (220) is screwed together with the outside thread (120) in a wider angle-of-rotation range.

2. The screw connector according to Claim 1, wherein the first connecting element (100, 100a, 100b) and the second connecting element (200, 200a, 200b) are each embodied as complementary halves of a common Luer connector.

3. The screw connector according to Claim 2, wherein the first lumen (110, 110a, 110b) is designed as the female half of the Luer connector and the second lumen (210, 210a, 210b) is designed as the male half of the Luer connector.

4. The screw connector according to one of Claims 1 to 3, wherein the first formation (140) is a local elevation on the core diameter of the outside thread (120) between two neighbouring thread flanks, and the second formation (240) is a local clearance on the thread flank of the inside thread (220).

5. The screw connector according to Claim 4, wherein the local clearance is designed as a groove running substantially in the axial direction of the inside thread (220) in a thread flank of the inside thread (220), and the local elevation is designed as a rib running substantially between two neighbouring thread flanks of the outside thread (120) in the axial direction of the outside thread (120).

6. The screw connector according to Claim 1, wherein the first connecting element (100, 100a, 100b) and the second connecting element (200, 200a, 200b) are each configured to set a half-open sterilization position of the screw connector for a gas sterilization and/or steam sterilization of the medical hose system in screw connection of the inside thread (220) to the outside thread (120) in the at least one predetermined second angle-of-rotation range.

7. A medical hose system having a screw connector according to one of Claims 1 to 6, wherein the medical hose system has a blood hose set for the extracorporeal blood treatment having an arterial patient hose and a venous patient hose, and the patient's end of the arterial patient hose (411) and the patient's end of the venous patient hose (421) are each designed as a second connecting element (200, 200a, 200b).

8. The medical hose system according to Claim 7, wherein the medical hose system has a three-way connector (300) with three connected lumens, wherein at least two of the three connected lumens are designed respectively as the first connecting element (100, 100a, 100b).

9. The medical hose system according to Claim 8, configured for short-circuiting the patient's end of the arterial patient hose (411) and the patient's end of the venous patient hose (421) with the three-way connector (300).

10. The medical hose system according to Claim 8, wherein the end of the arterial patient hose opposite the patient's end of the arterial patient hose (411) and the end of the venous patient hose opposite the patient's end of the venous patient hose (421) are connected to a blood cassette for the extracorporeal blood treatment.

11. The medical hose system according to Claim 10, wherein the medical hose system is packaged in a closed sterile wrapping and the patient's end of the arterial patient hose (411) and the patient's end of the venous patient hose (421) are connected to the three-way connector (300) in the half-open sterilization position.

12. Use of a screw connector according to one of Claims 1 to 6 for gas sterilization and/or steam sterilization of a medical hose system according to one of Claims 7 to 11.

## Revendications

1. Connecteur à vis pour système tubulaire médical, comportant au moins un premier élément de connexion (100,100a, 100b) doté d'un premier lumen (110,110a,110b) et d'un filetage extérieur (120) et au moins un second élément de connexion (200,200a,200b) doté d'un second lumen (210,210a,210b) et d'un écrou chapeau déformable élastiquement (230,230a,230b) doté d'un filetage intérieur (220), le filetage intérieur (220) et le filetage extérieur (120) étant configurés pour être vissés l'un à l'autre, le filetage extérieur présentant au moins une première excroissance (140) et le filetage intérieur au moins une seconde excroissance (240) et le filetage intérieur (220) et le filetage extérieur (120) étant configurés pour être vissés mutuellement sous déformation élastique de l'écrou chapeau (230,230a,230b) au moyen de la première excroissance (140) dans au moins une première plage d'angle de rotation prédéfinie sans que la première excroissance (140) s'enclenche dans la seconde excroissance (240) et le filetage intérieur (220) et le filetage extérieur (120) et la première excroissance (140) et la seconde excroissance (240) étant configurés pour enclencher la première excroissance (140) dans la seconde excroissance (240) lorsque le filetage intérieur (220) et le filetage extérieur (120) sont vissés dans une seconde plage d'angle de rotation prédéfinie,
**caractérisé en ce que**
le premier élément de connexion (100,100a,100b) et le second élément de connexion (200,200a,200b) sont respectivement configurés pour, lors du vissage du filetage intérieur (220) avec le filetage extérieur (120) dans l'au moins une seconde plage d'angle de rotation prédéfinie, être étanches au gaz entre un environnement extérieur du connecteur à vis et un intérieur constitué par le premier lumen (110, 110a, 110b) et le second lumen (210, 210a, 210b) et que le premier élément de connexion (100,100a,100b) et le second élément de connexion (200,200a,200b) sont respectivement configurés pour, lors du vissage du vissage du filetage intérieur (220) avec le filetage extérieur (120) dans une autre plage d'angle de rotation, établir une étanchéité de manière étanche au gaz et/ou au liquide entre l'environnement extérieur du connecteur à vis et l'intérieur du connecteur à vis.

2. Connecteur à vis selon la revendication 1, dans lequel le premier élément de connexion (100,100a,100b) et le second élément de connexion (200,200a,200b) sont respectivement réalisés sous forme de moitiés complémentaires d'un connecteur Luer commun.

3. Connecteur à vis selon la revendication 2, dans lequel le premier lumen (110,110a,110b) est réalisé sous forme de moitié femelle du connecteur Luer et le second lumen (210,210a,210b) sous forme de moitié mâle du connecteur Luer.

4. Connecteur à vis selon une des revendications 1 à 3, dans lequel la première excroissance (140) est un rehaussement local sur le diamètre de noyau du filetage extérieur (120) entre deux flancs filetés voisins et la seconde excroissance (240) est un dégagement local sur le flanc fileté du filetage intérieur (220).

5. Connecteur à vis selon la revendication 4, dans lequel le dégagement local est réalisé sous forme d'une orientée sensiblement dans le sens axial du filetage intérieur (220) dans un flanc fileté du filetage intérieur (220) et le rehaussement local est réalisé sous forme d'une nervure orientée sensiblement dans le sens axial du filetage extérieur (120) entre deux flancs filetés voisins du filetage extérieur (120).

6. Connecteur à vis selon la revendication 1, dans lequel le premier élément de connexion (100,100a,100b) et le second élément de connexion (200,200a,200b) sont respectivement configurés pour, lors du vissage du filetage intérieur (220) avec le filetage extérieur (120) établir, dans l'au moins une seconde plage d'angle de rotation prédéfinie, une position de stérilisation semi-ouverte du connecteur à vis pour une stérilisation au gaz et/ou à la vapeur du système tubulaire médical.

7. Système tubulaire médical comportant un connecteur à vis selon une des revendications 1 à 6, ce système tubulaire médical présentant un jeu de tuyaux à sang pour le traitement sanguin extracorporel comportant un tuyau artériel pour le patient et un tuyau veineux pour le patient et l'extrémité située du côté du patient du tuyau artériel pour le patient (411) et l'extrémité située du côté du patient du tuyau veineux pour le patient (421) étant réalisées respectivement sous forme d'un second élément de connexion (200,200a,200b).

8. Système tubulaire médical selon la revendication 7, dans lequel le système tubulaire médical présente un connecteur à trois distributions (300) doté de trois lumens connectés, au moins deux des trois lumens connectés se présentant respectivement sous forme du premier élément de connexion (100,100a,100b).

9. Système tubulaire médical selon la revendication 8, configuré pour court-circuiter l'extrémité située du côté du patient du tuyau artériel pour le patient (411) et l'extrémité située du côté du patient du tuyau veineux pour le patient (421) avec le connecteur à trois distributions (300).

10. Système tubulaire médical selon la revendication 8, dans lequel l'extrémité du tuyau artériel pour le patient opposée à l'extrémité située du côté du patient du tuyau artériel pour le patient (411) et l'extrémité du tuyau veineux pour le patient opposée à l'extrémité située du côté du patient du tuyau veineux pour le patient (421) sont connectées par une cassette à sang pour le traitement sanguin extracorporel.

11. Système tubulaire médical selon la revendication 10, dans lequel le système tubulaire médical est emballé de manière stérile dans une enveloppe fermée et l'extrémité située du côté du patient du tuyau artériel pour le patient (411) et l'extrémité située du côté du patient du tuyau veineux pour le patient (421) sont connectées, dans la position de stérilisation semi-ouverte, avec le connecteur à trois distributions (300).

12. Utilisation d'un connecteur à vis selon une des revendications 1 à 6 pour la stérilisation au gaz et/ou la stérilisation à la vapeur d'un système tubulaire médical selon une des revendications 7 à 11.
